# EUROPEAN PATENT SPECIFICATION

(11) **EP 2 222 229 B1**
(45) Date of publication and mention of the grant of the patent: **20.02.2013**
(21) Application number: 08850942.7
(22) Date of filing: 24.10.2008
(51) Int. Cl.: B01L 3/00

(54) **DEVICE FOR BIOCHEMICAL PROCESSING AND ANALYSIS OF A SAMPLE**
VORRICHTUNG ZUR BIOCHEMISCHEN AUFBEREITUNG UND ANALYSE EINER PROBE
DISPOSITIF DE TRAITEMENT BIOCHIMIQUE ET D'ANALYSE D'UN ÉCHANTILLON

(30) Priority: 12.11.2007 SE 0702489
(43) Date of publication of application: 01.09.2010
(73) Proprietor: LifeAssays AB (Publ), 223 70 Lund (SE)
(72) Inventor: KRIZ, Dario, S-243 95 Höör (SE); KRIZ, Kirstin, S-243 95 Höör (SE)
(74) Representative: Jakobsson, Jeanette Helene
(86) International application number: PCT/SE2008/051206
(87) International publication number: WO 2009/064241

(56) References cited:
- WO-A1-01/13795
- WO-A1-79/01131
- WO-A2-00/69389
- DE-C1- 10 105 753
- US-A- 3 811 326
- US-A- 4 935 020
- US-A- 5 651 940
- US-A- 5 658 531
- KRIZ K. ET AL: 'Detection of C-reactive protein utilizing magnetic permeability detection based immunoassays' ANAL. CHEM. vol. 77, no. 18, September 2005, pages 5920 - 5924, XP003024968

## Description

### Field of the Invention

The present invention relates to a disposable device for biochemical processing and analysis of a measured sample volume of a liquid sample. The invention is especially intended to be used for qualitative and quantitative biochemical analysis of body fluids (inter alia blood and urine) in near patient measurements but can also be used for analysis of other liquid samples in industrial process control, quality control as well as research and laboratory work.

### Background Art

A large number of near patient analyses are performed every day in hospitals, in primary health care and at home. In a frequent method, a measured sample volume of the patient's body fluid (for instance blood, plasma, urine, sweat, tears, lymph, amniotic fluid, cerebrospinal fluid and faeces) is collected in a capillary tube and transferred to a container, after which it is exposed to various specific reagents with which the body fluid reacts. The final quantitative or qualitative chemical analysis is performed by means of an optical detector in a transparent cuvette or on a measuring surface. The devices (for instance QuikRead manufactured by Axis Shield A/S, Norway) which are based on a manual method imply that the sample volume and reagent solutions may be spilt on people or work surfaces, resulting in health and environmental hazards. There is also a risk of incorrect analytical results due to laboratory mishandling. The devices (for instance Afinion manufactured by Axis Shield A/S, Norway) which are based on automated methods reduce the above-mentioned health and environmental hazards and also the risk of incorrect analytical results, but this is done by a costly and complex technical solution.

The specific reagents that are used are of the type biochemically (that is biologically and chemically) reactive substances, which may consist of monoclonal antibody, polyclonal antibody, enzyme, inorganic oxidising agents, inorganic reducing agents, metal ions, metal ion complexes, proteins, hormones, complementary factors, bacteria, cells, virus, fungi, yeast, spores, phages, cell organelles, peptides, DNA, RNA, coagulation inhibiting substances, cell lysing agents, antibiotics, tenside and active detergents.

After the body fluid having reacted with one or more specific reagents, this biological or chemical event is transformed into a physical change (optical, electric, radioactive or magnetic), which can be perceived by a detector. Optical detectors are popular especially in established immunoassay technologies that are used for near patient analyses. Optical detectors measure, inter alia, changes of the absorption of light, light scattering, fluorescence, polarisation, and require transparent cuvettes with transparent liquid sample contents. This results in the drawback that the liquid sample frequently has to be biochemically processed in several steps before it reaches the transparent cuvette or measuring surface. Electric detectors must be in direct contact with the liquid sample and therefore are sensitive to disturbing substances such as ascorbic acid in the body fluid. Radioactive detectors are rare in near patient analyses since they are a danger to people and environment. Magnetic detectors measure, inter alia, magnetic permeability and have the advantage that they allow quick and easy detection of the contents in non-transparent cuvettes which are allowed to contain non-transparent fluid, suspension, and capillary tubes. Such a magnetic detector is disclosed in SE9502902-1, US6,110,660 and Larsson K. et al. Analusis 27, p78 1999.

The present invention solves the above described problems in a new and effective way by offering the user a manually operable disposable device to provide leakage-free biochemical processing and analysis of a measured sample volume of a liquid sample with an eliminated risk of contamination of people and environment and a minimised risk of incorrect measured values without using instruments with automatic preparation of samples.

The above-mentioned commercially available devices and documents SE9502902-1 (Dario Kriz, 1995) US6,110,660 (Dario Kriz, 1995) and Larsson K. et al. (Analusis 27, p78, 1999) describe prior art devices and methods that are used for chemical processing and analysis of a measured sample volume of a liquid sample. However, said devices and methods do not contain a thin pierceable membrane through which an arm-fixed capillary tube passes and fits tightly around the arm after the insertion of the capillary tube. The present invention enables a manually operable disposable device to provide leakage-free biochemical processing and analysis of a measured sample volume of a liquid sample with an eliminated risk of contamination of people and environment and a minimised risk of incorrect measured values due to reagent losses related to leakage of liquid without using instruments with automatic preparation of samples and without necessitating a negative pressure or an injection mechanism in the inventive device.

Other prior art techniques comprise a liquid sample collecting device according to WO 79/01131 (Robert Turner and Reginald Holman, 1978). This device comprises a pierceable flexible membrane which is penetrated by a capillary tube. The membrane fits tightly around the capillary tube, of which each end is on an associated side of the membrane. To allow the sample volume in the capillary tube to be drawn into the device there is a negative pressure in the device. The present invention does not require a negative pressure since both ends of the capillary tube pass the membrane and the sample volume is shaken out of the capillary tube. Furthermore the device according to WO 79/01131 doe not contain any substances for biochemical processing and analysis.

Other prior art techniques comprise a sample collecting device according to US5,833,630 (Bernd Kloth, 1997). This device comprises a capillary tube and substances for biochemical processing and analysis. The device does not comprise a pierceable membrane which is penetrated by the capillary tube. The capillary tube is positioned in a duct in a stopper which is placed on the device. The capillary tube is pressed into (but not through) the stopper by means of a cap, the generated positive pressure forcing the sample volume out of the capillary tube and down into the device. Since the device does not have a pierceable membrane and requires manual exchange of the stopper (from a stopper without capillary tube to one with capillary tube), there is a risk of some spilling of the reagent solution of the device, which results in incorrect measured results. Moreover the emptying of the capillary tube will not be as quick and effective as in the present invention since the forced liquid movement through the capillary tube in the present invention cleans the capillary tube without leaving any residues of adsorbed sample solution.

Other prior art techniques comprise a device for handling organic body fluids according to SE451942 (Bengt-Inge Brodén, 1986). This device comprises a capillary tube but no substances for biochemical processing and analysis. The device does not comprise a pierceable membrane which is penetrated by the capillary tube. The capillary tube is positioned in a duct in a stopper which is placed on the device. Air is forced through the capillary tube by means of a sprayer, the generated positive pressure forcing the sample volume out of the capillary tube and down into the device. The device does not contain any substances for chemical processing and analysis and is designed to reduce the risk of contamination caused by spilling of body fluid samples. Furthermore the emptying of the capillary tube will not be as quick and effective as with the present invention since the forced liquid movement through the capillary tube in the present invention cleans the capillary tube without leaving any residues of adsorbed sample solution.

Other prior art techniques comprise a combination reagent and test device for analysing liquids according to US5,888,826 (Roy Ostgaard et el., 1997). This device comprises a pierceable membrane and substances for biochemical processing and analysis. The device does not comprise a capillary tube and is not designed for manual handling (mixing of sample solution and reagent) but requires advanced automatic instruments for function.

Other prior art techniques comprise a disposable device for analysing liquids according to US6,319,209 (Dario Kriz, 1999). This device comprises a capillary tube and substances for biochemical processing and analysis. Since the device does not have a pierceable membrane and requires manual turning of a stopper (from one without to one with capillary tube) there is a risk of some spilling of the reagent solution of the device, which results in incorrect measured results. Moreover the emptying of the capillary tube will not be as quick and effective as in the present invention since the forced liquid movement through the capillary tube in the present invention cleans the capillary tube without leaving any residues of adsorbed sample solution.

### Summary of the Invention

Thus the present invention relates to a device, characterised in that it comprises a sealed vessel (1), which contains at least one thin pierceable membrane (2), through which a capillary tube (3) fixed to an arm (9) can pass into the vessel. When the arm (9) has inserted the capillary tube (3) into the vessel (1), the opening in the membrane (2) is sealed by the rear part of the arm (9). The sealed vessel (1) contains, in addition to a liquid (6), at least one biochemically active substance (4) and/or at least one marker substance (5) and/or a sediment of carrier particles (7) depending on which quantitative or qualitative chemical analysis is to be performed.

The invention also relates to a method in which a device according to the invention is used to empty, by shaking (both manually and automatically), the contents of the capillary tube (3) into the vessel (1) to begin the biochemical processing and analysis of the measured sample volume of a liquid sample. The invention further concerns a method in which a device according to the invention after shaking is placed in an instrument comprising a detector for reading of physical changes for the purpose of performing qualitative or quantitative analyses of various biological or chemical substances.

### Brief Description of the Drawings

Fig. 1 illustrates the device according to the present invention with an intact pierceable membrane (2) and the capillary tube (3) attached to the arm (9).
Fig. 2 illustrates the device according to the present invention in a pushed-together state, in which the pierceable membrane (2) has been pierced by the capillary tube (3) and the pierced hole has been sealed by the arm (9). The capillary tube (3) is placed in the sealed vessel (1).

### Detailed Description of the Invention

According to one aspect of the invention, the device is characterised in that the sealed vessel (1) has a volume in the range 0.1-250 ml and that it contains a liquid (6), and that the thickness of the thin pierced membrane (2) is in the range 0.01-5 mm, and that the thin pierced membrane (2) is attached in a circular opening adapted to fit tightly against the arm (9) and has a diameter in the range 0.5-5 mm, and that the capillary tube (3) has a length in the range 1-30 mm, and that the capillary tube (3) has an outer diameter in the range 0.2-3 mm, and that a filled capillary tube (3) may contain a measured sample volume in the range 0.1-200 µl, and that the collar (8), which facilitates the insertion of the capillary tube (3), has a length in the range 1-20 mm.

According to another aspect, the device is characterised in that the sealed vessel (1) contains one or more biochemically reactive substances (4), which may consist of monoclonal antibody, polyclonal antibody, enzyme, inorganic oxidising agents, inorganic reducing agents, metal ion, metal ion complex, protein, hormone, complementary factor, bacterium, cell, virus, fungus, yeast, spore, phage, cell organelle, peptide, DNA, RNA, coagulation inhibiting substance, cell lysing agents, antibiotics, tenside, active detergent, EDTA, adenosine 5' diphosphate, ristocetin, arachidonic acid, thrombin, epinephrine, platelet activator factor or thrombin receptor agonist peptide (TRAP). The biochemically reactive substances (4) that are used depend on what analysis is to be performed and have generally known and well-documented functions, which comprise, for example, binding to the biological or chemical substance that is to be determined, catalytic conversion of the biological or chemical substance that is to be determined, stabilisation of the contents in the sealed vessel (1) so as to allow long-term storage, stabilisation of the biological or chemical substance that is to be determined once it is inserted in the sealed vessel (1) so that correct analytical results can be obtained, deactivation of disturbing biological or chemical substances that may disturb the measurement, and cell lysis or release of the biological or chemical substance that is to be determined so as to obtain correct analytical results.

According to another aspect, the device is characterised in that the sealed vessel (1) contains one or more marker substances (5), which may consist of magnetically influenceable reagents, such as superparamagnetic nanoparticles, antibody-derivatised superparamagnetic nanoparticles, protein-derivatised superparamagnetic nanoparticles, polymer-derivatised superparamagnetic nanoparticles, peptide-derivatised superparamagnetic nanoparticles, DNA- or RNA-derivatised superparamagnetic nanoparticles, carbohydrate-derivatised superparamagnetic nanoparticles,
or alternatively that the marker substance (5) consists of an optical, electric or radioactive reagent based on antibodies, enzymes, inorganic oxidising agents, inorganic reducing agents, metal ions and metal ion complexes, proteins, peptides, polymers, carbohydrates, complementary factors, blood coagulation factors, hormones, bacteria, cells, viruses, fungi, yeast, spores, phages, cell organelles, DNA, RNA, coagulation inhibiting substances, antibiotics, tenside and active detergent. The marker substances (5) that are used depend on what analysis is to be performed and have generally known and well-documented functions which comprise interaction with the biological or chemical substance that is to be determined and generation of a quantifiable physical change (optical, electric, radioactive or magnetic), which can be perceived by a detector.

According to yet another aspect, the device is characterised in that the contents in the sealed vessel (1) have a relative magnetic permeability (µᵣ), which is increased relative to water and which is in the range 1.00001 < µᵣ < 10.

According to a further aspect, the device is characterised in that the carrier particles (7) have antibodies or alternatively lectines, or alternatively proteins, or alternatively peptides, or alternatively DNA or RNA, or alternatively nothing bound to their surface and have a diameter between 0.5 micrometer and 5 mm and can consist of hydrophilic silica, hydrophobic silica, glass, silicon dioxide, carbohydrates, ion exchangers, polymers, ceramic materials, proteins, bacteria. The carrier particles (7) that are used depend on what analysis is to be performed and have generally known and well-documented functions, which comprise binding and enriching of the biological or chemical substance with which the marker substance (5) is associated and which thus accumulates a quantifiable physical change (optical, electric, radioactive or magnetic) in the bottom sediment which can be perceived by a detector.

According to another aspect, the device is characterised in that said liquid (6) consists of an aqueous solution containing at least one acidity regulating agent, such as 0.1 M sodium phosphate pH 7, and at least one ionic strength adjusting agent, such as 0.1 M sodium chloride. The liquid (6) that is used depends on what analysis is to be performed and has generally known and well-documented functions, which comprise, for example, dissolution of proteins, salts and sample liquid for an analysis to be performed. Moreover, the liquid (6) satisfies the requirements in respect of salt content and pH (acidity) which are placed on the matrix by the biochemically reactive substances (4), the marker substances (5) and the carrier particles (7) due to their function and which influence stability, cell-cell interactions, cell-ligand interactions, antibody-antigen interactions, binding, catalytic capacity and enzymatic activity.

According to a further aspect, the device is characterised in that it is fitted with a capillary holder which comprises an arm (9) of plastic, in which a capillary tube (3) of glass is mounted, or alternatively that the arm (9) is a unit which also has the form of a capillary tube (3). The capillary tubes (3) that are used depend on the sample volume that is to be measured and have generally known and well-documented functions, which comprise chemical material compatibility with the biological or chemical substance and the liquid sample that is to be analysed.

The device is characterised in that it is fitted with a capillary holder which comprises an arm (9), said arm (9) having a conically shaped thickening of the outer diameter or that it has a collar (10), by which the opening in said thin pierceable membrane is sealed after the insertion of the capillary tube (3).

According to a further aspect, the device is characterised in that it is fitted with a capillary holder which comprises an arm (9), said arm (9) having an air vent (11) in the form of a hole (with the diameter 0.2-5 mm) or alternatively in the form of a gap (having the width 0.2-5 mm and the length 1-20 mm) which extends parallel to the capillary tube and through which pressure equalisation occurs so as to allow filling of said capillary tube (3).

According to another aspect, the device is characterised in that it is fitted with a capillary holder which also comprises a cap (12), which facilitates the handling of the capillary tube (3) and the height of which in the range 1-20 mm is adjusted to the length of said arm (9) and the location of the capillary tube (3) on said arm (9) so as to allow the insertion of the capillary tube (3) into said sealed vessel (1) in a predetermined and reproducible manner, implying that the conically shaped thickening of the outer diameter or alternatively the collar (10) forms, with the opening in said thin pierceable membrane, a hermetic and/or leakage-free seal after the insertion of the capillary tube (3).

According a further aspect, the device according to the invention is characterised in that the vessel (1) comprises at least one internal wing (13), which facilitates the emptying, by shaking (both manually and automatically), the contents of the capillary tube (3) into the vessel (1) to begin the biochemical processing and analysis of the measured sample volume of a sample. The emptying of the capillary content is facilitated by increased fluid turbulences caused by the at least one internal wing (13). This results in that the mixing of the liquid and sample in the device is facilitated.

According to one aspect, the internal wing (13) has a length and width in the range of 0.2-5 mm and a thickness in the range of 0.2-5 mm.

According to a further aspect, the device according to the invention is characterised in that the material of which said sealed vessel (1), said thin pierceable membrane (2), said capillary holder and said capillary tube (3) are made is one or a combination of the following materials, such as polymers, for instance Delrin, Perspex, POM, polyvinylchloride, polyvinyl fluoride, Teflon, polyamide, polyacetal, nylon, polyethylene, polycarbonate, polystyrene, and polypropylene, or alternatively a material such as glass, rubber, wood, paper and metal.

According to a further aspect, the device according to the inventions is characterised in that the material of which said sealed vessel (1) and/or said thin pierceable membrane (2) are made is a non-transparent material, for instance black polymer, for the purpose of protecting light-sensitive biochemically reactive substances (4) from being detrimentally affected by light in long time storage of the device. The use of a non-transparent material is compatible with magnetic detectors (and not with optical detectors) since their measuring process is not disturbed.

According to a further aspect, the device according to the invention is characterised in that said samples consist of body fluids such as blood, plasma, urine, sweat, tears, lymph, amniotic fluid, cerebrospinal fluid and faeces.

According to another aspect, the device according to the invention is characterised in that measured volumes of said sample, when consisting of faeces, can be manually pressed into the cavity of the capillary tube (3) without the use of capillary forces.

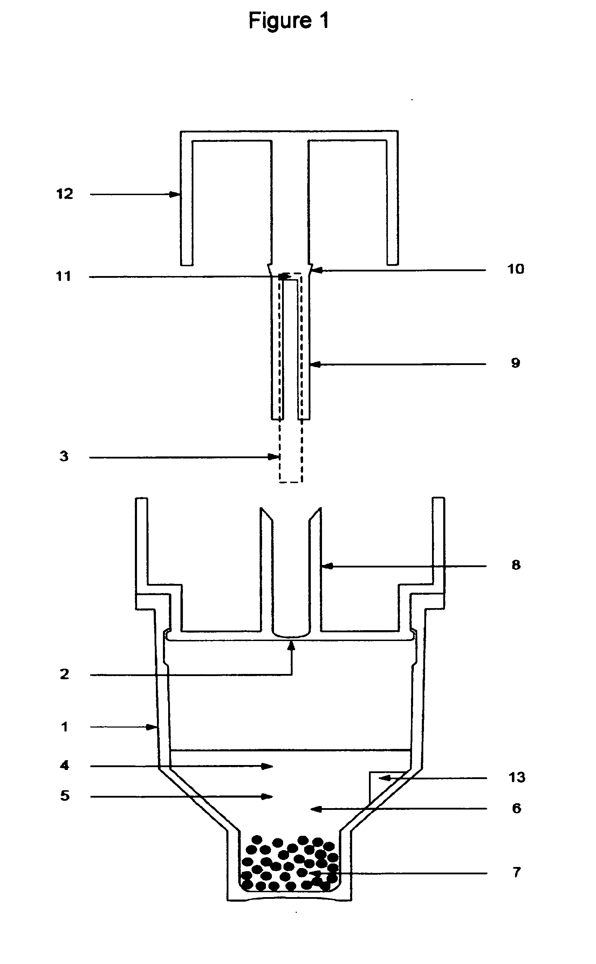

Fig. 1 is a view (on a scale of 1:3, that is 30 mm in the figure corresponds to 10 mm in real life) of the device according to the present invention. The device according to Fig. 1 comprises an intact pierceable membrane (2) of polypropylene and the capillary tube (3) of glass is attached to the arm (9) of polycarbonate. The sealed vessel (1) of polypropylene contains a liquid (6) consisting of 0.1 M sodium phosphate buffer pH 7.0 with 0.1 M sodium chloride, and a biochemically active substance (4) (EDTA) which prevents blood coagulation, and a marker substance (5) consisting of antiCRP monoclonal antibodies coupled to superparamagnetic nanoparticles, and a bottom sediment of carrier particles (7) consisting of antiCRP polyclonal antibodies coupled to silica particles with a diameter 15-40 µm, and a collar (8) of polypropylene. Further the device according to Fig. 1 comprises a capillary holder with the arm (9), which fixes the capillary tube (3) and the cap (12) of polycarbonate. The arm (9) also comprises a collar (10) of polycarbonate and an air vent (11).

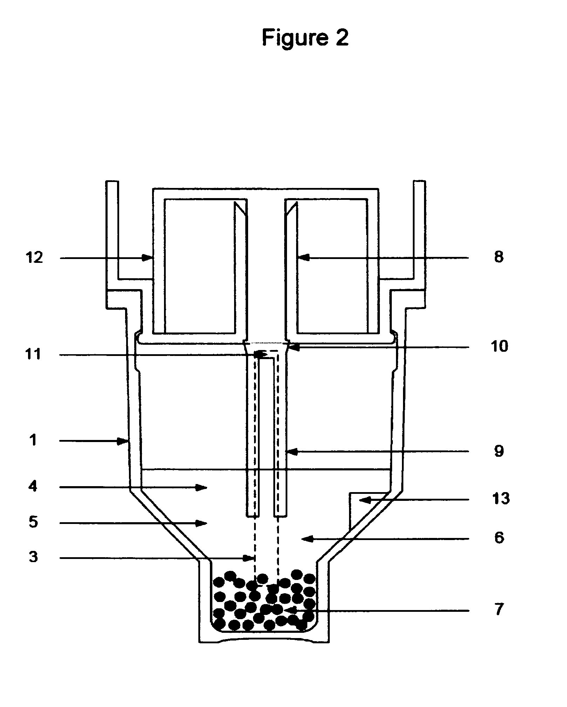

Fig. 2 illustrates the device according to the invention in a pushed-together state, the pierceable membrane (2) being pierced by the capillary tube (3) and the pierced hole being sealed by the collar (10) on the arm (9). The capillary tube (3) is placed in the sealed vessel (1).

The device according to the invention may advantageously be used together with a magnetic detector by the device being placed in or in the immediate vicinity of an electric coil for detection of magnetic permeability µ, or alternatively relative magnetic permeability µᵣ, or alternatively relative magnetic susceptibility (µᵣ -1).

The device according to the invention may advantageously be used together with an optical detector by the device being placed in the vicinity of a light source (for instance bulb, light emitting diode or laser) for measuring optical phenomena such as the changes of light absorption, light scattering, fluorescence and polarisation.

The device according to the invention may advantageously be used for detection of on the one hand chemical substances with high magnetic permeability and, on the other, chemical substances having approximately the same relative magnetic permeability as water, that is µᵣ = 1, such as glucose, C-reactive protein (CRP and hsCRP), albumin, cystatin C, hemoglobin (Hb and HbA1C), myoglobin, troponin (I and T), CK-MB, creatine kinase (CK), d-dimer, BNP, proBNP, NT-proBNP, prothrombin, APTT, HCG, LH, FSH, PSA, TSH, T3, T4, AFP, CEA, lipoproteins (LDL and HDL), triglycerides, cholesterol, antibodies, Streptococcus A, Heliobacter Pylori, Salmonella, Chlamydia, Giardia, cholera, hepatitis (A, B and C) adenoviruses, rotaviruses, proteins, hormones, complementary factors, blood coagulation factors, cell-ligand interactions, cell-cell interactions, platelet aggregations, bacteria, cells, viruses, fungi, yeast, spores, phages, cells, cell organelles, DNA, RNA, which all require interaction with one or more magnetically influenceable reagents.

The device according to the invention may advantageously be used for a qualitative and respectively quantitative near patient one-time analysis (socalled Point-of-Care analysis) of glucose, C-reactive protein (CRP and hsCRP), albumin, cystatin C, hemoglobin (Hb and HbA1C), myoglobin, troponin (I and T), CK-MB, creatine kinase (CK), d-dimer, BNP, proBNP, NT-proBNP, prothrombin, APTT, HCG, LH, FSH, PSA, TSH, T3, T4, AFP, CEA, lipoproteins (LDL and HDL), triglycerides, cholesterol, antibodies, Streptococcus A, Heliobacter Pylori, Salmonella, Chlamydia, Giardia, cholera, hepatitis (A, B and C) adenoviruses, rotaviruses, proteins, hormones, complementary factors, blood coagulation factors, cell-ligand interactions, cell-cell interactions, platelet aggregations, bacteria, cells, viruses, fungi, yeast, spores, phages, cells, cell organelles, DNA, RNA, in various types of body fluids such as blood, plasma, urine, sweat, tears, lymph, cerebrospinal fluid and faeces.

The device according to the invention may advantageously be used for qualitative and respectively quantitative analysis of glucose, C-reactive protein (CRP and hsCRP), albumin, cystatin C, hemoglobin (Hb and HbA1C), myoglobin, troponin (I and T), CK-MB, creatine kinase (CK), d-dimer, BNP, proBNP, NT-proBNP, prothrombin, APTT, HCG, LH, FSH, PSA, TSH, T3, T4, AFP, CEA, lipoproteins (LDL and HDL), triglycerides, cholesterol, antibodies, Streptococcus A, Heliobacter Pylori, Salmonella, Chlamydia, Giardia, cholera, hepatitis (A, B and C) adenoviruses, rotaviruses, proteins, hormones, complementary factors, blood coagulation factors, cell-ligand interactions, cell-cell interactions, platelet aggregations, bacteria, cells, viruses, fungi, yeast, spores, phages, cells, cell organelles, DNA, RNA, in various types of industrial process control, quality control, research and laboratory work.

The device according to the invention may advantageously be marked with information such as analytical identification data and production lot number, last day of consumption, and date of production.

## Claims

1. A device for biochemical processing and analysis of a measured sample volume of a sample, wherein the device consists of a sealed vessel (1),
the device comprises at least one thin pierceable membrane (2) through which a capillary tube (3) containing said measured sample volume of a sample can pass into said sealed vessel (1),
said sealed vessel (1) contains at least one biochemically reactive substance (4), and
said sealed vessel (1) contains a liquid (6),
said device comprising a capillary holder with an arm (9), in which said capillary tube (3) is to be mounted and by means of which said capillary tube (3) is to be inserted through said thin pierceable membrane (2) into said sealed vessel (1),
**characterised in that** said arm (9) has a conically shaped thickening of the outer diameter or that it has a collar (10) by means of which the opening of said thin pierceable membrane is sealed after the insertion of the capillary tube (3).

2. A device as claimed in claim 1, **characterised in that** said vessel (1) comprises at least one internal wing (13) for facilitating the mixing of the liquid (6) and the sample in the device.

3. A device as claimed in claim 2, **characterised in that** said at least one internal wing (13) has a length and width in the range of 0.2-5 mm and a thickness in the range of 0.2-5 mm.

4. A device as claimed in any one of claims 1-3, **characterised in that** said sealed vessel (1) contains at least one marker substance (5) and/or carrier particles (7) forming a bottom sediment.

5. A device as claimed in any one of claims 1-4, **characterised in that** said thin pierceable membrane (2) is surrounded by a collar (8) on the outside of the device.

6. A device as claimed in any one of claims claim 1-5, **characterised in that** said arm (9) comprises a mounted capillary tube (3), or alternatively that the arm (9) is a unit which also comprises the shape of a capillary tube (3).

7. A device as claimed in any one of claims 1-6, **characterised in that** said arm (9) has an air vent (11) in the form of a hole or alternatively in the form of a gap which extends parallel to the capillary tube (3) and through which pressure equalisation occurs so as to allow filling of said capillary tube (3).

8. A device as claimed in any one of claims 1-7, **characterised in that** said capillary holder also comprises a cap (12).

9. A device as claimed in any one of claims 1-8, **characterised in that** the material of which said sealed vessel (1), said thin pierceable membrane (2), said capillary holder and said capillary tube (3) is made is one or a combination of the following materials consisting of transparent/non-transparent polymers, Delrin, Perspex, POM, polyvinylchloride, polyvinyl fluoride, Teflon, polyamide, polyacetal, nylon, polyethylene, polycarbonate, polystyrene, and polypropylene, or alternatively a material chosen from glass, rubber, wood, paper and metal.

10. A device as claimed in any one of claims 1-9, **characterised in that** said sample consist of body fluids such as blood, plasma, urine, sweat, tears, lymph, amniotic fluid, cerebrospinal fluid and faeces.

11. A device as claimed in any one of claims 1-10, **characterised in that** said biochemically reactive substance (4) consists of monoclonal antibody, polyclonal antibody, enzyme, inorganic oxidising agents, inorganic reducing agents, metal ion, metal ion complex, protein, hormone, complementary factor, bacterium, cell, virus, fungus, yeast, spore, phage, cell organelle, peptide, DNA, RNA, coagulation inhibiting substance, cell lysing agents, antibiotics, tenside, active detergent, EDTA, adenosine 5' diphosphate, ristocetin, arachidonic acid, thrombin, epinephrine, platelet activator factor or thrombin receptor agonist peptide (TRAP).

12. A device as claimed in any one of claims 1-11, **characterised in that** said liquid (6) consists of an aqueous solution containing at least.one acidity regulating agent, such as 0.1 M sodium phosphate pH 7, and at least one ionic strength adjusting agent, such as 0.1 M sodium chloride.

13. A device as claimed in any one of claims 4-12, **characterised in that** said marker substance (5) consists of a magnetically influenceable reagent, such as superparamagnetic nanoparticles, antibody-derivatised superparamagnetic nanoparticles, protein-derivatised superparamagnetic nanoparticles, polymer-derivatised superparamagnetic nanoparticles, peptide-derivatised superparamagnetic nanoparticles, DNA- or RNA-derivatised superparamagnetic nanoparticles, carbohydrate-derivatised superparamagnetic nanoparticles,
or alternatively that the marker substance (5) consists of an optical, electric or radioactive reagent based on antibodies, enzymes, inorganic oxidising agents, inorganic reducing agents, metal ions and metal ion complexes, proteins, peptides, polymers, carbohydrates, complementary factors, blood coagulation factors, hormones, bacteria, cells, viruses, fungi, yeast, spores, phages, cell organelles, DNA, RNA, coagulation inhibiting substances, antibiotics, tenside and active detergent.

14. A device as claimed in any one of claims 4-13, **characterised in that** said carrier particles (7) have antibodies or alternatively lectins, or alternatively proteins, or alternatively peptides, or alternatively DNA or RNA, or alternatively nothing bound to their surface and have a diameter between 0.5 micrometer and 5 mm and can consist of hydrophilic silica, hydrophobic silica, glass, silicon dioxide, carbohydrates, ion exchangers, polymers, ceramic materials, proteins, bacteria.

15. A device as claimed in any one of claims 1-14, **characterised in that** measured volumes of said sample, when consisting of faeces, can be manually pressed into the cavity of the capillary tube (3) without the use of capillary forces.

16. A method in which the device as claimed in any one of claims 1-15 is placed in or in the immediate vicinity of an electric coil for detection of magnetic permeability µ, or alternatively relative magnetic permeability µᵣ, or - alternatively relative magnetic susceptibility (µᵣ -1).

17. A method in which the device as claimed in any one of claims 1-15, by the biochemical processing of said liquid sample in said sealed vessel, is used for a qualitative and respectively quantitative near patient one-time analysis of glucose, C-reactive protein (CRP and hsCRP), albumin, cystatin C, hemoglobin (Hb and HbA1C), myoglobin, troponin (1 and T), CK-MB, creatine kinase (CK), d-dimer, BNP, proBNP, NT-proBNP, prothrombin, APTT, HCG, LH, FSH, PSA, TSH, T3, T4, AFP, CEA, lipoproteins (LDL and HDL), triglycerides, cholesterol, antibodies, Streptococcus A, Heliobacter Pylori, Salmonella, Chlamydia, Giardia, cholera, hepatitis (A, B and C) adenoviruses, rotaviruses, proteins, hormones, complementary factors, blood coagulation factors, cell-ligand interactions, cell-cell interactions, platelet aggregations, bacteria, cells, viruses, fungi, yeast, spores, phages, cells, cell organelles, DNA, RNA, in various types of body fluids such as blood, plasma, urine, sweat, tears, lymph, cerebrospinal fluid and faeces.

18. A method in which the device as claimed in any one of claims 1-15 by the biochemical processing of said liquid sample in said sealed vessel, is used for qualitative and respectively quantitative analysis of glucose, C-reactive protein (CRP and hsCRP), albumin, cystatin C, hemoglobin (Hb and HbA1C), myoglobin, troponin (1 and T), CK-MB, creatine kinase (CK), d-dimer, BNP, proBNP, NT-proBNP, prothrombin, APTT, HCG, LH, FSH, PSA, TSH, T3, T4, AFP, CEA, lipoproteins (LDL and HDL), triglycerides, cholesterol, antibodies, Streptococcus A, Heliobacter Pylori, Salmonella, Chlamydia, Giardia, cholera, hepatitis (A, B and C) adenoviruses, rotaviruses, proteins, hormones, complementary factors, blood coagulation factors, cell-ligand interactions, cell-cell interactions, platelet aggregations, bacteria, cells, viruses, fungi, yeast, spores, phages, cells, cell organelles, DNA, RNA.

## Patentansprüche

1. Vorrichtung zur biochemischen Aufbereitung und Analyse eines abgemessenen Probenvolumens einer Probe, wobei
die Vorrichtung aus einem versiegelten Behälter (1) besteht,
die Vorrichtung mindestens eine dünne durchstechbare Membran (2) umfasst, durch welche ein Kapillarrohr (3), welches das abgemessene Probenvolumen einer Probe enthält, in den versiegelten Behälter (1) führbar ist,
der versiegelte Behälter (1) mindestens eine biochemisch reaktive Substanz (4) enthält und
der versiegelte Behälter (1) eine Flüssigkeit (6) enthält,
wobei die Vorrichtung eine Kapillarenhalterung mit einem Arm (9) umfasst, in welcher das Kapillarrohr (3) zu montieren ist und mittels dessen das Kapillarrohr (3) durch die dünne durchstechbare Membran (2) in den versiegelten Behälter (1) einzuführen ist,
**dadurch gekennzeichnet, dass** der Arm (9) eine konisch geformte Verdickung des äußeren Durchmessers aufweist oder dass er einen Hülse (10) aufweist, mittels welcher die Öffnung der dünnen durchstechbaren Membran nach dem Einführen des Kapillarrohrs (3) versiegelt wird.

2. Vorrichtung nach Anspruch 1, **dadurch gekennzeichnet, dass** der Behälter (1) mindestens einen inneren Flügel (13) umfasst, um das Vermischen der Flüssigkeit (6) mit der Probe in der Vorrichtung zu erleichtern.

3. Vorrichtung nach Anspruch 2, **dadurch gekennzeichnet, dass** der mindestens eine innere Flügel (13) eine Länge und Breite im Bereich von 0,2 mm bis 5 mm und eine Dicke im Bereich von 0,2 mm bis 5 mm aufweist.

4. Vorrichtung nach einem der Ansprüche 1 bis 3, **dadurch gekennzeichnet, dass** der versiegelte Behälter (1) mindestens eine Marker-Substanz (5) und/oder Trägerpartikel (7) enthält, welche ein Bodensediment bilden.

5. Vorrichtung nach einem der Ansprüche 1 bis 4, **dadurch gekennzeichnet, dass** die dünne durchstechbare Membran (2) an der Außenseite der Membran von einer Hülse (8) umgeben ist.

6. Vorrichtung nach einem der Ansprüche 1 bis 5,
**dadurch gekennzeichnet, dass** der Arm (9) ein montiertes Kapillarrohr (3) umfasst, oder dass es sich alternativ bei dem Arm (9) um eine Einheit handelt, welche ebenfalls die Form eines Kapillarrohrs (3) umfasst.

7. Vorrichtung nach einem der Ansprüche 1 bis 6, **dadurch gekennzeichnet, dass** der Arm (9) eine Entlüftungsöffnung (11) in Form eines Lochs oder alternativ in Form eines Spalts aufweist, welche sich parallel zu dem Kapillarrohr (3) erstreckt und durch welche ein Druckausgleich erfolgt, um das Befüllen des Kapillarrohrs (3) zu ermöglichen.

8. Vorrichtung nach einem der Ansprüche 1 bis 3, **dadurch gekennzeichnet, dass** die Kapillarenhalterung außerdem eine Abdeckung (12) umfasst.

9. Vorrichtung nach einem der Ansprüche 1 bis 8,
**dadurch gekennzeichnet, dass** es sich bei dem Material, aus welchem der versiegelte Behälter (1), die dünne durchstechbare Membran (2), die Kapillarenhalterung und das Kapillarrohr (3) hergestellt sind, um eines oder eine Kombination der folgenden Materialien handelt, welche aus transparenten/nicht transparenten Polymeren bestehen: Delrin, Perspex, POM, Polyvinylchlorid, Polyvinylfluorid, Teflon, Polyamid, Polyacetal, Nylon, Polyethylen, Polycarbonat, Polystyrol und Polypropylen, oder alternativ um ein Material handelt, welches aus Glas, Gummi, Holz, Papier und Metall ausgewählt ist.

10. Vorrichtung nach einem der Ansprüche 1 bis 9,
**dadurch gekennzeichnet, dass** die Probe aus Körperfluiden, wie z.B. Blut, Plasma, Urin, Schweiß, Tränen, Lymphe, Fruchtwasser, Zerebrospinalfluid und Faezes, besteht.

11. Vorrichtung nach einem der Ansprüche 1 bis 10,
**dadurch gekennzeichnet, dass** die biochemisch reaktive Substanz (4) aus monoklonalem Antikörper, polyklonalem Antikörper, Enzym, anorganischem Oxidationsmittel, anorganischem Reduktionsmittel, Metallionen, Metallionenkomplexen, Protein, Hormonen, Komplementfaktoren, Bakterien, Zellen, Viren, Pilzen, Hefe, Sporen, Phagen, Zellorganellen, Peptid, DNA, RNA, gerinnungshemmender Substanz, zelllysierenden Mitteln, Antibiotika, Tensid, aktivem Detergens, EDTA, Adenosin-5'-diphosphat, Ristotecin, Arachidonsäure, Thrombin, Epinephrin, thrombozytenaktivierendem Faktor oder Thrombin-Rezeptor-Agonist-Peptid (TRAP) besteht.

12. Vorrichtung nach einem der Ansprüche 1 bis 11,
**dadurch gekennzeichnet, dass** die Flüssigkeit (6) aus einer wässrigen Lösung besteht, welche mindestens ein die Azidität regulierendes Mittel, z.B. 0,1 M Natriumphosphat pH 7, und mindestens ein die Ionenstärke regulierendes einstellendes Mittel enthält, z.B. 0,1 M Natriumchlorid.

13. Vorrichtung nach einem der Ansprüche 4 bis 12,
**dadurch gekennzeichnet, dass** die Marker-Substanz (5) aus einem magnetisch beeinflussbaren Reagens besteht, z.B. aus superparamagnetischen Nanopartikeln, Antikörper-derivatisierten superparamagnetischen Nanopartikeln, Proteinderivatisierten superparamagnetischen Nanopartikeln, Polymer-derivatisierten superparamagnetischen Nanopartikeln, Peptid-derivatisierten superparamagnetischen Nanopartikeln, DNA- oder RNA-derivatisierten superparamagnetischen Nanopartikeln, Kohlenhydrat-derivatisierten superparamagnetischen Nanopartikeln,
oder dass die Marker-Substanz (5) alternativ aus einem optischen, elektrischen oder radioaktiven Reagens auf der Basis von Antikörpern, Enzymen, anorganischen Oxidationsmitteln, anorganischen Reduktionsmitteln, Metallionen und Metallionenkomplexen, Proteinen, Peptiden, Polymeren, Kohlenhydraten, Komplementfaktoren, Blutgerinnungsfaktoren, Hormonen, Bakterien, Zellen, Viren, Pilzen, Hefe, Sporen, Phagen, Zellorganellen, Peptid, DNA, RNA, gerinnungshemmenden Substanzen, Antibiotika, Tensid und aktivem Detergens besteht.

14. Vorrichtung nach einem der Ansprüche 4 bis 13,
**dadurch gekennzeichnet, dass** die Trägerpartikel (7) an ihre Oberfläche gebunden Antikörper oder alternativ Lectine oder alternativ Proteine oder alternativ Peptide oder alternativ DNA oder RNA oder alternativ nichts an ihre Oberfläche gebundenes aufweisen und einen Durchmesser von 0,5 Mikrometern bis zu 5 mm aufweisen und z.B. aus hydrophilem Silica, hydrophobem Silica, Glas, Siliciumdioxid, Kohlenhydraten, Ionenaustauschern, Polymeren, keramischen Materialien, Proteinen, Bakterien bestehen.

15. Vorrichtung nach einem der Ansprüche 1 bis 14,
**dadurch gekennzeichnet, dass** abgemessene Volumen der Probe, wenn sie aus Faezes besteht, ohne die Anwendung von Kapillarkräften manuell in den Hohlraum des Kapillarrohrs gepresst werden können.

16. Verfahren, bei welchem die Vorrichtung nach einem der Ansprüche 1 bis 15 zum Erfassen der magnetischen Permeabilität µ oder alternativ der relativen magnetischen Permeabilität µᵣ oder alternativ der relativen magnetischen Suszeptibilität (µᵣ-1) in oder in der unmittelbaren Nachbarschaft einer elektrischen Spule angeordnet wird.

17. Verfahren, bei welchem die Vorrichtung nach einem der Ansprüche 1 bis 15 durch die biochemische Aufbereitung der flüssigen Probe in dem versiegelten Behälter für eine patientennahe einmalige qualitative bzw. quantitative Analyse auf Glucose, C-reaktives Protein (CRP und hsCRP), Albumin, Cystatin C, Hämoglobin (Hb und HbA1 C), Myoglobin, Troponin (I und T), CK-MB, Kreatinkinase (CK), d-Dimer, BNP, proBNP, NT-proBNP, Prothrombin, APTT, HCG, LH, FSH, PSA, TSH, T3, T4, AFP, CEA, Lipoproteine (LDL und HDL), Triglyceride, Cholesterin, Antikörper, Streptococcus A, Helicobacter pylori, Salmonellen, Chlamydien, Giardien,
Cholera, Hepatitis (A, B und C), Adenoviren, Rotaviren, Proteine, Hormone, Komplementfaktoren, Blutgerinnungsfaktoren, Zell-Liganden-Wechselwirkungen, Zell-Zell-Wechselwirkungen, Thrombozytenaggregationen, Bakterien, Zellen, Viren, Pilzen, Hefe, Sporen, Phagen, Zellen, Zellorganellen, DNA, RNA in verschiedenen Arten von Körperfluiden, wie z.B. Blut, Plasma, Urin, Schweiß, Tränen, Lymphe, Zerebrospinalfluid und Faezes, verwendet wird.

18. Verfahren, bei welchem die Vorrichtung nach einem der Ansprüche 1 bis 15 durch die biochemische Aufbereitung der flüssigen Probe in dem versiegelten Behälter für eine qualitative bzw. quantitative Analyse auf Glucose, C-reaktives Protein (CRP und hsCRP), Albumin, Cystatin C, Hämoglobin (Hb und HbA1 C), Myoglobin, Troponin (1 und T), CK-MB, Kreatinkinase (CK), d-Dimer, BNP, proBNP, NT-proBNP, Prothrombin, APTT, HCG, LH, FSH, PSA, TSH, T3, T4, AFP, CEA, Lipoproteine (LDL und HDL), Triglyceride, Cholesterin, Antikörper, Streptococcus A, Heliobacter pylori, Salmonellen, Chlamydien, Giardien, Cholera, Hepatitis (A, B und C), Adenoviren, Rotaviren, Proteine, Hormone, Komplementfaktoren, Blutgerinnungsfaktoren, Zell-Liganden-Wechselwirkungen, Zell-Zell-Wechselwirkungen, Thrombozytenaggregationen, Bakterien, Zellen, Viren, Pilzen, Hefe, Sporen, Phagen, Zellen, Zellorganellen, DNA, RNA verwendet wird.

## Revendications

1. Dispositif de traitement biochimique et d'analyse d'un volume d'échantillon mesuré à partir d'un échantillon, dans lequel
le dispositif est constitué d'un récipient scellé (1),
le dispositif comprend au moins une membrane mince perforable (2) qu'un tube capillaire (3) contenant ledit volume d'échantillon mesuré à partir d'un échantillon peut traverser pour pénétrer dans ledit récipient scellé (1),
ledit récipient scellé (1) contient au moins une substance (4) réactive sur le plan biochimique et
ledit récipient scellé (1) contient un liquide (6),
ledit dispositif comportant un support pour capillaire pourvu d'un bras (9), dans lequel ledit tube capillaire (3) doit être monté et au moyen duquel ledit tube capillaire (3) doit être inséré à travers ladite membrane mince perforable (2) dans ledit récipient scellé (1),
**caractérisé en ce que** ledit bras (9) possède un épaississement conique du diamètre extérieur ou un collet (10) au moyen duquel l'ouverture de ladite membrane mince perforable est scellée après l'insertion du tube capillaire (3).

2. Dispositif selon la revendication 1, **caractérisé en ce que** ledit récipient (1) comprend au moins une aile interne (13) pour faciliter le mélange du liquide (6) et de l'échantillon dans le dispositif.

3. Dispositif selon la revendication 2, **caractérisé en ce que** ladite ou lesdites ailes internes (13) possèdent une longueur et une largeur dans la plage de 0,2 à 5 mm et une épaisseur dans la plage de 0,2 à 5 mm.

4. Dispositif selon l'une quelconque des revendications 1 à 3, **caractérisé en ce que** ledit récipient scellé (1) contient au moins une substance de marquage (5) et/ou des particules supports (7) formant un sédiment de fond.

5. Dispositif selon l'une quelconque des revendications 1 à 4, **caractérisé en ce que** ladite membrane mince perforable (2) est entourée d'un col (8) à l'extérieur du dispositif.

6. Dispositif selon l'une quelconque des revendications 1 à 5, **caractérisé en ce que** ledit bras (9) comprend un tube capillaire (3) monté, ou bien, en variante, **en ce que** le bras (9) est une unité comprenant également la forme d'un tube capillaire (3).

7. Dispositif selon l'une quelconque des revendications 1 à 6, **caractérisé en ce que** ledit bras (9) possède un évent d'air (11) sous la forme d'un trou ou bien, en variante, sous la forme d'un espace s'étendant parallèlement au tube capillaire (3) et à travers lequel une égalisation de la pression s'effectue afin de permettre le remplissage dudit tube capillaire (3).

8. Dispositif selon l'une quelconque des revendications 1 à 7, **caractérisé en ce que** ledit support pour tube capillaire comprend également un bouchon (12).

9. Dispositif selon l'une quelconque des revendications 1 à 8, **caractérisé en ce que** le matériau dont sont constitués ledit récipient scellé (1), ladite membrane mince perforable (2), ledit support pour tube capillaire et ledit tube capillaire (3) est formé d'un ou d'une combinaison des matériaux suivants parmi : polymères transparents/non transparents, Delrin, Perspex, POM, polychlorure de vinyle, polyfluorure de vinyle, Teflon, polyamide, polyacétal, nylon, polyéthylène, polycarbonate, polystyrène et polypropylène, ou bien, en variante, un matériau choisi parmi le verre, le caoutchouc, le bois, le papier et le métal.

10. Dispositif selon l'une quelconque des revendications 1 à 9, **caractérisé en ce que** ledit échantillon consiste en des fluides corporels tels que sang, plasma, urine, sueur, larmes, lymphe, liquide amniotique, liquide céphalorachidien et selles.

11. Dispositif selon l'une quelconque des revendications 1 à 10, **caractérisé en ce que** ladite substance (4) réactive sur le plan biochimique consiste en anticorps monoclonal, anticorps polyclonal, enzyme, agents oxydants inorganiques, agents réducteurs inorganiques, ion métallique, complexe d'ion métallique, protéine, hormone, facteur du complément, bactérie, cellule, virus, champignon, levure, spore, phage, organite cellulaire, peptide, ADN, ARN, substance anticoagulante, agents de lyse cellulaire, antibiotiques, tensioactif, détergent actif, EDTA, adénosine-5'-diphosphate, ristocétine, acide arachidonique, thrombine, adrénaline, facteur d'activation plaquettaire ou peptide agoniste du récepteur de la thrombine (TRAP).

12. Dispositif selon l'une quelconque des revendications 1 à 11, **caractérisé en ce que** ledit liquide (6) consiste en une solution aqueuse contenant au moins un agent régulateur d'acidité, tel que le phosphate de sodium 0,1 M pH 7, et au moins un agent d'ajustement de la force ionique, tel que le chlorure de sodium 0,1 M.

13. Dispositif selon l'une quelconque des revendications 4 à 12, **caractérisé en ce que** ladite substance de marquage (5) consiste en un réactif magnétiquement influençable, tel que des nanoparticules superparamagnétiques, des nanoparticules superparamagnétiques modifiées par des anticorps, des nanoparticules superparamagnétiques modifiées par des protéines, des nanoparticules superparamagnétiques modifiées par des polymères, des nanoparticules superparamagnétiques modifiées par des peptides, des nanoparticules superparamagnétiques modifiées par de l'ADN ou de l'ARN, des nanoparticules superparamagnétiques modifiées par des glucides,
ou bien, en variante, **en ce que** la substance de marquage (5) consiste en un réactif optique, électrique ou radioactif à base d'anticorps, enzymes, agents oxydants inorganiques, agents réducteurs inorganiques, ions métalliques et complexes d'ions métalliques, protéines, peptides, polymères, glucides, facteurs du complément, facteurs de coagulation sanguine, hormones, bactéries, cellules, virus, champignons, levures, spores, phages, organites cellulaires, ADN, ARN, substances anticoagulantes, antibiotiques, tensioactif et détergents actifs.

14. Dispositif selon l'une quelconque des revendications 4 à 13, **caractérisé en ce que** lesdites particules supports (7) ont des anticorps ou bien des lectines, ou bien des protéines, ou bien des peptides, ou bien de l'ADN ou de l'ARN lié à leur surface ou bien aucun élément lié à leur surface et ont un diamètre entre 0,5 micromètre et 5 mm et peuvent être constituées de silice hydrophile, silice hydrophobe, verre, dioxyde de silicium, glucides, échangeurs d'ions, polymères, matériaux céramiques, protéines, bactéries.

15. Dispositif selon l'une quelconque des revendications 1 à 14, **caractérisé en ce que** des volumes mesurés dudit échantillon, lorsqu'il s'agit de selles, peuvent être pressés manuellement dans la cavité du tube capillaire (3) sans recours aux forces capillaires.

16. Procédé dans lequel le dispositif selon l'une quelconque des revendications 1 à 15 est placé dans ou à proximité immédiate d'une bobine électrique pour la détection de la perméabilité magnétique p, ou bien de la perméabilité magnétique relative µᵣ, ou bien de la susceptibilité magnétique relative (µᵣ-1).

17. Procédé dans lequel le dispositif selon l'une quelconque des revendications 1 à 15, par le traitement biochimique dudit échantillon liquide dans ledit récipient scellé, est employé pour l'analyse qualitative et respectivement quantitative ponctuelle, à proximité du patient de : glucose, protéine C-réactive (CRP et CRP-HS), albumine, cystatine C, hémoglobine (Hb et HbA1 C), myoglobine, troponine (I et T), CK-MB, créatine kinase (CK), d-dimère, BNP, pro-BNP, NT-proBNP, prothrombine, APTT, HCG, LH, FSH, PSA, TSH, T3, T4, AFP,CEA, lipoprotéines (LDL et HDL), triglycérides, cholestérol, anticorps, streptocoque A, *Heliobacter pylori, Salmonella, Chlamydia, Giardia,* choléra, hépatite (A, B et C), adénovirus, rotavirus, protéines, hormones, facteurs du complément, facteurs de coagulation sanguine, interactions cellule-ligand, interactions cellule-cellule, agrégations plaquettaires, bactéries, cellules, virus, champignons, levures, spores, phages, cellules, organites cellulaires, ADN, ARN, dans différents types de fluides corporels tels sang, plasma, urine, sueur, larmes, lymphe, liquide céphalorachidien et selles.

18. Procédé dans lequel le dispositif selon l'une quelconque des revendications 1 à 15, par le traitement biochimique dudit échantillon liquide dans ledit récipient scellé, est employé pour l'analyse qualitative et respectivement quantitative de : glucose, protéine C-réactive (CRP et CRP-HS), albumine, cystatine C, hémoglobine (Hb et HbA1 C), myoglobine, troponine (I et T), CK-MB, créatine kinase (CK), d-dimère, BNP, pro-BNP, NT-proBNP, prothrombine, APTT, HCG, LH, FSH, PSA, TSH, T3, T4, AFP,CEA, lipoprotéines (LDL et HDL), triglycérides, cholestérol, anticorps, streptocoque A, *Heliobacter pylori, Salmonella, Chlamydia, Giardia,* choléra, hépatite (A, B et C), adénovirus, rotavirus, protéines, hormones, facteurs du complément, facteurs de coagulation sanguine, interactions cellule-ligand, interactions cellule-cellule, agrégations plaquettaires, bactéries, cellules, virus, champignons, levures, spores, phages, cellules, organites cellulaires, ADN, ARN.
